(19) Europäisches Patentamt
European Patent Office
Office européen des brevets

(11) Veröffentlichungsnummer : **0 548 017 A1**

# (12) EUROPÄISCHE PATENTANMELDUNG

(21) Anmeldenummer : **92810971.9**

(22) Anmeldetag : **09.12.92**

(51) Int. Cl.$^5$ : **C07D 417/12,** A61K 31/425

(30) Priorität : **18.12.91 CH 3750/91**

(43) Veröffentlichungstag der Anmeldung :
**23.06.93 Patentblatt 93/25**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Missbach, Martin, Dr.**
**Cristalinweg 4**
**CH-4310 Rheinfelden (CH)**

## (54) Neue Thiazole.

(57) Neue Thiazole der Formel I

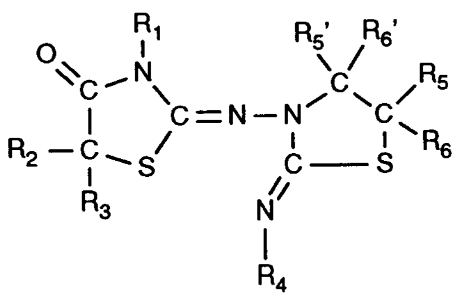

(I)

worin $R_1$ und $R_4$ Niederalkyl, Niederalk-2-en-1-yl oder auch Niederalk-2-in-1-yl bedeuten, $R_2$ und $R_3$ unabhängig voneinander Wasserstoff oder Niederalkyl bedeuten oder gemeinsam für Niederalkyliden stehen, und $R_5$ und $R_5$ jeweils für Wasserstoff oder Niederalkyl stehen oder gemeinsam Oxo darstellen, und $R_5'$ und $R_6'$ die gleiche Bedeutung von $R_5$ und $R_6$ haben, mit der Massgabe, dass mindestens einer der Substituentenpaare $R_5$ und $R_6$ oder $R_5'$ und $R_6'$ gemeinsam für Oxo stehen und ihre Salze, Verfahren zur Herstellung der genannten Verbindungen, diese enthaltende pharmazeutische Präparate und ihre Verwendung als Arzneimittelwirkstoffe.

EP 0 548 017 A1

Jouve, 18, rue Saint-Denis, 75001 PARIS

Die Erfindung betrifft neue Thiazole der Formel I

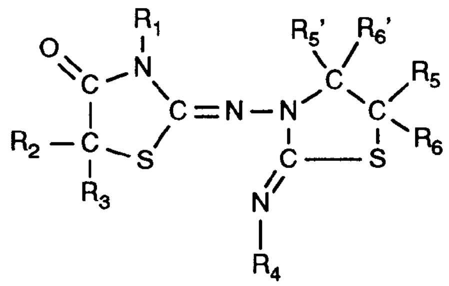

(I)

worin $R_1$ und $R_4$ Niederalkyl, Niederalk-2-en-1-yl oder auch Niederalk-2-in-1-yl $R_2$ und $R_3$ unabhängig voneinander Wasserstoff oder Niederalkyl bedeuten oder gemeinsam für Niederalkyliden stehen, und $R_5$ und $R_6$ jeweils für Wasserstoff oder Niederalkyl stehen oder gemeinsam Oxo darstellen, und $R_6$' und $R_6$' die gleiche Bedeutung von $R_5$ und $R_6$ haben, mit der Massgabe, dass mindestens einer der Substituentenpaare $R_5$ und $R_6$ oder $R_5$' und $R_6$' gemeinsam für Oxo stehen und ihre Salze, Verfahren zur Herstellung der genannten Verbindungen, diese enthaltende pharmazeutische Präparate und ihre Verwendung als Arzneimittelwirkstoffe.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalk-2-en-1-yl ist beispielsweise $C_3$-$C_5$-Alk-2-en-1-yl, wie insbesondere Allyl oder Methallyl.

Niederalk-2-in-1-yl ist beispielsweise $C_3$-$C_5$-Alk-2-in-1-yl, wie insbesondere Prop-2-in-1-yl oder auch But-2-in-1-yl.

Niederalkyl ist beispielsweise $C_1$-$C_4$-Alkyl, wie Methyl, Ethyl, Propyl oder Butyl.

Niederalkyliden ist beispielsweise $C_1$-$C_4$-Alkyliden, wie insbesondere Methylen.

Pharmazeutisch verwendbare Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate. Salze von Verbindungen der Formel I sind beispielsweise deren Säureadditionssalze, beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Die Verbindungen der Formel I und ihre pharmazeutisch verwendbaren Salze weisen wertvolle pharmakologische Eigenschaften auf. Insbesondere weisen sie ausgeprägte antiarthritische Eigenschaften auf. Diese können *in vivo* beispielsweise am Modell der Adjuvans-Arthritis der Ratte nach I. Wiesenberg et al. Clin. Exp. Immunol. 78, 245 (1989) in Dosierungen ab etwa 0,1 bis 0,3 mg/kg p.o. oder i.p. nachgewiesen werden.

Die Verbindungen der Formel I und ihre pharmazeutisch verwendbaren Salze können deshalb zur Behandlung von Erkrankungen des rheumatoiden Formenkreises eingesetzt werden. Zu diesen gehören insbesondere die rheumatoide Arthritis, die juvenile Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie Verbindungen der Formel I, worin $R_1$ $C_3$-$C_5$-Alk-2-en-1-yl oder $C_3$-$C_5$-Alk-2-in-1-yl und $R_4$ $C_1$-$C_4$-Alkyl, $C_3$-$C_5$-Alk-2-en-1-yl, oder $C_3$-$C_5$-Alk-2-in-1-yl bedeutet, $R_2$ und $R_3$ unabhängig voneinander Wasserstoff oder $C_1$-$C_4$-Alkyl bedeuten oder gemeinsam $C_1$-$C_4$-Alkyliden darstellen und $R_6$ und $R_6$ bzw. $R_6$' und $R_6$' jeweils unabhängig voneinander Wasserstoff oder $C_1$-$C_4$-Alkyl oder gemeinsam Oxo darstellen, mit der Massgabe, dass mindestens einer der Substituentenpaare $R_6$ und $R_6$ oder $R_6$' und $R_6$' gemeinsam für Oxo stehen, und ihre Salze, insbesondere ihre pharmazeutisch verwendbaren Salze.

Die Erfindung betrifft vor allem Verbindungen der Formel I, worin der Rest $R_1$ $C_3$-$C_5$-Alk-2-en-1-yl, wie Allyl oder Methallyl oder $C_3$-$C_5$-Alk-2-in-1-yl, wie Prop-2-in-2-yl, und $R_4$ $C_1$-$C_4$-Alkyl, wie Methyl, oder $C_3$-$C_5$-Alk-2-en-1-yl, wie Allyl oder Methallyl, bedeutet, $R_2$ und $R_3$ beide Wasserstoff oder gleiche $C_1$-$C_4$-Alkylgruppen, wie

Methyl, bedeuten oder gemeinsam $C_1$-$C_4$-Alkyliden, wie Methylen darstellen und $R_5$ und $R_6$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_6$' und $R_6$' gemeinsam für Oxo stehen, und ihre Salze, insbesondere ihre pharmazeutisch verwendbare Salze.

Die Erfindung betrifft insbesondere Verbindungen der Formel I, worin $R_1$ Allyl oder Methallyl, $R_2$ und $R_3$ beide Wasserstoff oder Methyl bedeuten, $R_4$ für Methyl, Allyl oder Methallyl stehen und $R_6$ und $R_6$ bzw. $R_6$' und $R_6$' jeweils Wasserstoff oder gemeinsam Oxo darstellen, mit der Massgabe, dass mindestens einer der Substituentenpaare $R_6$ und $R_6$ oder $R_6$' und $R_6$' gemeinsam für Oxo stehen, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der erfindungsgemäss bereitgestellten Verbindungen ist dadurch gekennzeichnet, dass man einen Verbindung der Formel II

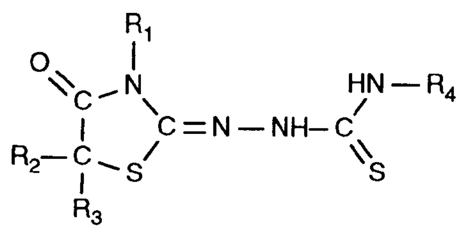

(II)

mit einer entsprechenden Verbindung der Formel III

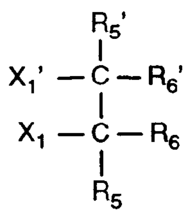

(III)

worin $X_1$ und $X_1$' jeweils ein reaktionsfähiges verestertes Hydroxy bedeuten, und $R_5$, $R_6$, $R_5$' und $R_6$' die oben angegebenen Bedeutungen haben, in Gegenwart eines basischen Kondensationsmittels unter nachfolgendem Erwärmen kondensiert.

Die Kondensation erfolgt in Gegenwart eines basischen Kondensationsmittels, wie einer tertiären organischen Base, wie eines Triniederalkylamins, von Hünig'scher Base oder einer organischen Stickstoffbase, wie Pyridin oder Chinolin, und/oder anfangs unter Kühlen, vorteilhaft im Temperaturbereich von etwa +15 bis -70°C, beispielsweise von 0 bis -60°C, insbesondere von -40 bis -60°C und nachfolgendem Erwärmen auf eine Temperatur von +20 bis +60°, insbesondere von +30 bis +40°.

Die Verbindungen der Formel II und auf an sich bekannten Methoden beruhende Verfahren zu Ihrer Herstellung sind bekannt und beispielsweise in GB-1,325,061, US-4,697,020, DE-2,405,395, oder DE-2,632,745 als Zwischenprodukte für die Herstellung von tumorhemmenden Arzneimittelwirkstoffen beschrieben.

In Ausgangsstoffen der Formel III kommen als abspaltbare Reste $X_1$ bzw. $X'_1$ beispielsweise reaktionsfähig veresterte Hydroxygruppen, insbesondere Halogen, wie Chlor oder Brom, in Betracht. Verbindungen der Formel III sind bekannt und beispielsweise Oxalylchlorid oder auch Halogenacetylhalogenide, wie z.B. Bromacetylchloride oder auch -bromid, welche käuflich zu erwerben sind.

Die Kondensation von Verbindungen der Formel II mit Verbindungen der Formel III erfolgt in üblicher Weise, vorteilhaft in einem inerten Lösungsmittel, wie z.B. eines aliphatischen Halogen-Kohlenwasserstoffes, wie in Dichlormethan, oder eines aliphatischen oder cycloaliphatischen Ethers, z.B. in Tetrahydrofuran oder auch Dioxan. In einer bevorzugten Ausführungsform der Verfahrensvariante a) setzt man beispielsweise anfangs bei einer Temperatur von -40 bis -60°C eine Verbindung der Formel II in Gegenwart von Triethylamin oder Pyridin und in Dichlormethan oder Tetrahydrofuran als Lösungsmittel mit einer Verbindung der Formel III, z.B. Oxalylchlorid oder einem Halogenacetylhalogenid, wie z.B. Bromacetylchlorid oder -bromid um und erwärmt anschliessend auf eine Temperatur von +20 bis + 60°, insbesondere von +30 bis +40°.

Die nach dem Verfahren erfindungsgemäss erhältlichen als Isomerengemisch vorliegenden Verbindungen der Formel I können gewünschtenfalls in die einzelnen Isomeren aufgetrennt werden und/oder erfindungsgemäss erhältliche freie Verbindungen in ein Salz oder ein erfindungsgemäss erhältliches Salz in eine freie Verbindung oder in ein anderes Salz überführt werden.

Verfahrensgemäss erhältliche Verbindungen können in üblicher Weise in andere Verbindungen der Formel I überführt werden.

Erhaltene Salze können in an sich bekannter Weise in die freien Verbindungen umgewandelt werden, z.B. durch Behandeln mit einer Base, wie einem Alkalimetallhydroxid , einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder n- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und

Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykol oder höhere Akanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykol oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Erfindung betrifft ebenfalls die Verwendung der Verbindungen der Formel II, vorzugsweise in Form von pharmazeutischen Präparaten. Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 5 mg bis etwa 1000 mg, insbesondere von etwa 10 mg bis etwa 200 mg zu veranschlagen. Diese kann auf auf einmal gegeben oder auf mehrere, z.B. 2 bis 4 Einzeldosen verteilt werden. Pharmazeutische Präparate in Dosiseinheitsform enthalten somit von etwa 5 mg bis etwa 250 mg, insbesondere von etwa 10 mg bis etwa 50 mg.

Die nachfolgen Beispiele dienen zur Illustration der Erfindung; Temperaturen sind in Celsiusgraden, Drucke in mbar angegeben.

Beispiel 1:

Zu einer Mischung von 4,9 g 3-Allyl-thiazolidin-2,4-dion-2-(4-methyl-3-thiosemicarbazon) und 6,2 ml Triethylamin in 100 ml absolutem Tetrahydrofuran werden unter Rühren bei -40° 1,9 ml Bromacetylbromid während 5 Minuten zugetropft und 15 Minuten bei derselben Temperatur gerührt. Die Suspension wird auf Raumtemperatur aufwärmen gelassen und anschliessend 30 Minuten bei 40° gerührt.

Nach dem Abziehen des Lösungsmittels wird das Gemisch zwischen Wasser und Methylenchlorid verteilt. Die wässrige Phase wird ein zweites Mal extrahiert und die vereinigten organischen Phasen über Natriumsulfat getrocknet und eingeengt. Aus kaltem Ethanol erhält man farblose Kristalle von 1-(3-Allyl-4-oxo-thiazolidin-2-yliden)-1-amino-2-methylimino-thiazolidin-5-on, Schmelzpunkt 100°.
$^1$H-NMR: 3.15 (s, 3H), 3.9 (2xs, 4H), 4.5 (d, 2H), 5.2-5.4 (m, 2H), 5.95 (m, 1H).

Beispiel 2:

In analoger Weise wie zu Beispiel 1 werden aus 6 g 3-Methallyl-5,5-dimethyl -thiazolidin-2,4-dion-2-(4-allyl-3-thiosemicarbazon) und 4,4 g Bromacetylbromid nach Umkristallisation aus Ethanol das 1-(3-Methallyl-5,5-dimethyl-4-oxo-thiazolidin-2-yliden)-1-amino-2-allylimino-thiazolidin-5-on erhalten, Schmelzpunkt 65°.
$^1$H-NMR: 1.65 (s, 6H), 1.8 (s, 3H), 3.8 (s, 2H), 3.95 (d, 2H), 4.45 (s, 2H), 4.85 (s, 1H), 4.95 (s, 1H), 5.1-5.25 (m, 2H), 5.9 (m, 1H).

Beispiel 3:

In analoger Weise wie zu Beispiel I aus 1,7 g 3-Methallyl-5,5-dimethylthiazolidin -2,4-dion-2-(4-methyl-3-thiosemicarbazon) und 1,2 g Bromacetylbromid nach Umkristallisation aus Ethanol das 1-(3-Methallyl-5,5-dimethyl-4-oxo-thiazolidin-2-yliden)-1-amino-2-methylimino-thiazolidin-5-on erhalten, Schmelzpunkt 135-138°.
$^1$H-NMR: 1.65 (s, 6H), 1.8 (s, 3H), 3.15 (s, 3H), 3.8 (s, 2H), 4.45 (s, 2H), 4.85 (s, 1H), 4.95 (s, 1H).

Beispiel 4:

In analoger Weise wie zu Beispiel 1 werden aus 2,0 g 3-Allylthiazolidin-2,4-dion-2-(4-allyl-3-thiosemicarbazon) und 0,72 ml Bromacetylbromid nach Umkristallisation aus Ethanol das 1-(3-Allyl-4-oxo-thiazolidin-2-yliden)-1-amino-2-allyliminothiazolidin -5-on erhalten, Schmelzpunkt 113-114°.
$^1$H-NMR: 3.85 (s, 4H), 3.95 (d, 2H), 4.5 (d 2H), 5.1 (d, 1H), 5.15-5.3 (d, 2H), 5.35 (d, 1H) 5. 85-6.0 (m, 2H).

Beispiel 5:

In analoger Weise wie zu Beispiel 1 werden aus 1,5 g 3-Propylthiazolidin-2,4-dion-2-(4-methyl-3-thiosemicarbazon) und 0,55 ml Bromacetylbromid nach Umkristallisation aus Ethanol das 1-(3-Propyl-4-oxo-thiazolidin-2-yliden)-1-amino-2-methylimino-thiazolidin-5-on erhalten, Schmelzpunkt 109-111 °.
$^1$H-NMR: 0.95 (t, 3H), 1.8 (dq, 2H), 3.15 (s, 3H), 3.8-3.9 (m, 6H).

Beispiel 6:

Tabletten, enthaltend je 10 mg Wirkstoff der Formel I, können wie folgt hergestellt werden:

| Zusammensetzung (10000 Tabletten) | |
|---|---|
| Wirkstoff | 100,0 g |
| Lactose | 450,0 g |
| Kartoffelstärke | 350,0 g |
| Gelatine | 10,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 50,0 mg Wirkstoffgehalt, die gewünschtenfalls mit Teilkerben zur feineren Anpassung der Dosierung versehen sein können.

Beispiel 7:

Gelatinesteckkapseln, enthaltend 20 mg Wirkstoff der Formel I, können z.B folgendermassen hergestellt werden:

| Zusammensetzung (für 1000 Kapseln) | |
|---|---|
| Wirkstoff | 20,0 g |
| Lactose | 240,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 300 mg der erhaltenen Formulierung in Gelatinesteckkapseln der Grösse 0 abgefüllt.

Beispiel 8:

Gelatinesteckkapseln, enthaltend 100 mg Wirkstoff der Formel I, können z.B. folgendermassen hergestellt werden:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 9:

Lacktabletten, enthaltend je 50 mg Wirkstoff der Formel I, können wie folgt hergestellt werden:

| Zusammensetzung (für 1000 Lacktabletten) | |
|---|---|
| Wirkstoff | 50,0 g |
| Lactose | 100,0 g |
| Maisstärke | 70,0 g |
| Talk | 10,0 g |
| Calciumstearat | 2,0 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 240 mg) verpresst und diese mit einer Lösung der Hydroxypropylmethylcellulose und des Schellacks in Methylenchlorid lackiert; Endgewicht der Lacktablette: 283 mg.

Beispiel 10:

Eine 0,2%-ige Injektions- oder Infusionslösung eines Wirkstoffs der Formel I, kann beispielsweise folgendermassen hergestellt werden:

| Zusammensetzung (für 1000 Ampullen) | |
|---|---|
| Wirkstoff | 5,0 g |
| Natriumchlorid | 22,5 g |
| Phosphatpuffer pH=7,4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 11:

1%-ige Wirkstoff der Formel I enthaltende Salbe (O/W-Emulsion), mit folgender Zusammensetzung:

| | |
|---|---|
| Wirkstoff | 1,0 g |
| Cethylalkohol | 3,0 g |
| Glycerin | 6,0 g |
| Methylparaben | 0,18 g |
| Propylparaben | 0,05 g |
| Arlacel 60 | 0,6 g |
| Tween 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

Beispiel 12:

1%-iges Gel, enthaltend als Wirkstoff eine Verbindung der Formel I, mit folgender Zusammensetzung:

| | |
|---|---|
| Wirkstoff | 1,0 g |
| Carbopol 934 P | 1,0 g |
| Glycerin | 3,0 g |
| Isopropanol | 25,0 g |
| Softigen® 767 | 0,2 g |
| Wasser entmin. q.s. ad | 100,0 g |

**Patentansprüche**

1. Eine Verbindung der Formel I

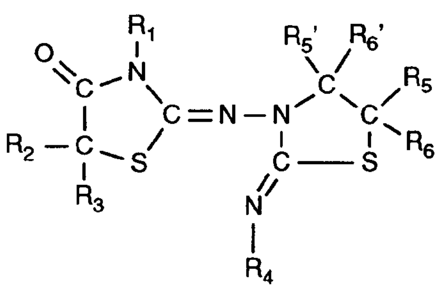

(I)

worin $R_1$ und $R_4$ Niederalkyl, Niederalk-2-en-1-yl oder auch Niederalk-2-in-1-yl bedeuten, $R_2$ und $R_3$ unabhängig voneinander Wasserstoff oder Niederalkyl bedeuten oder gemeinsam für Niederalkyliden stehen, und $R_5$ und $R_6$ jeweils für Wasserstoff oder Niederalkyl stehen oder gemeinsam Oxo darstellen, und $R_5$' und $R_6$' die gleiche Bedeutung von $R_5$ und $R_5$ haben, mit der Massgabe, dass mindestens einer der Substituentenpaare $R_5$ und $R_6$ oder $R_5$' und $R_5$' gemeinsam für Oxo stehen und Salze davon.

2. Eine Verbindung gemäss Anspruch 1 der Formel I, worin $R_1$ $C_3$-$C_5$-Alk-2-en-1-yl oder $C_3$-$C_5$-Alk-2-in-1-yl und $R_4$ $C_1$-$C_4$-Alkyl, $C_3$-$C_5$-Alk-2-en-1-yl oder $C_3$-$C_5$-Alk-2-in-1-yl bedeutet, $R_2$ und $R_3$ unabhängig voneinander Wasserstoff oder $C_1$-$C_4$-Alkyl bedeuten oder gemeinsam $C_1$-$C_4$-Alkyliden darstellen und $R_5$ und $R_6$ bzw. $R_5$' und $R_6$' jeweils unabhängig voneinander Wasserstoff oder $C_1$-$C_4$-Alkyl oder gemeinsam Oxo darstellen, mit der Massgabe, dass mindestens einer der Substituentenpaare $R_5$ und $R_6$ oder $R_5$' und $R_6$' gemeinsam für Oxo stehen, und Salze davon.

3. Eine Verbindung gemäss Anspruch 1 der Formel I, worin der Rest $R_1$ $C_3$-$C_5$-Alk-2-en-1-yl, wie Allyl oder Methallyl oder $C_3$-$C_5$-Alk-2-in-1-yl, wie Prop-2-in-2-yl, und $R_4$ $C_1$-$C_4$-Alkyl, wie Methyl, oder $C_3$-$C_5$-Alk-2-en-1-yl, wie Allyl oder Methallyl, bedeutet, $R_2$ und $R_3$ beide Wasserstoff oder gleiche $C_1$-$C_4$-Alkylgruppen, wie Methyl, bedeuten oder gemeinsam $C_1$-$C_4$-Alkyliden, wie Methylen darstellen und $R_5$ und $R_6$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_5$' und $R_5$' gemeinsam für Oxo stehen, und pharmazeutisch verwendbare Salze davon.

4. Eine Verbindung gemäss Anspruch 1 der Formel I, worin $R_1$ Allyl oder Methallyl, $R_2$ und $R_3$ beide Wasserstoff oder Methyl bedeuten, $R_4$ für Methyl, Allyl oder Methallyl stehen und $R_5$ und $R_5$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_5$' und $R_5$' gemeinsam für Oxo stehen, und pharmazeutisch verwendbare Salze davon.

5. 1-(3-Allyl-4-oxo-thiazolidin-2-yliden)-1-amino-2-methylimino-thiazolidin-5-on oder ein pharmazeutisch verwendbares Salz davon.

6. 1-(3-Methallyl-5,5-dimethyl-4-oxo-thiazolidin-2-yliden)-1-amino-2-allyliminothiazolidin-5-on oder ein pharmazeutisch verwendbares Salz davon.

7. 1-(3-Methallyl-5,5-dimethyl-4-oxo-thiazolidin-2-yliden)-1-amino-2-methyliminothiazolidin-5-on oder ein pharmazeutisch verwendbares Salz davon.

8. 1-(3-Allyl-4-oxo-thiazolidin-2-yliden)-1-amino-2-allylimino-thiazolidin-5-on oder ein pharmazeutisch verwendbares Salz davon.

9. 1-(3-Propyl-4-oxo-thiazolidin-2-yliden)-1-amino-2-methylimino-thiazolidin-5-on oder ein pharmazeutisch verwendbares Salz davon.

10. Verbindungen gemäss einem der Ansprüche 1 bis 9 zur Anwendung in einem Verfahren zur therapeutischen Behandlung des menschlichen oder tierischen Körpers.

11. Pharmazeutische Präparate, neben üblichen pharmazeutischen Hilfsstoffen als pharmazeutischen Wirkstoff enthaltend eine Verbindung gemäss einem der Ansprüche 1 bis 9 in freier Form oder in pharmazeutisch verwendbarer Salzform.

12. Die Verbindungen der allgemeinen Formel I gemäss Anspruch 1 als wirksame Mittel zur Behandlung von Erkrankungen des rheumatoiden Formenkreises.

13. Die Verwendung von Verbindungen der allgemeinen Formel I gemäss Anspruch 1 und üblichen Trägerstoffen zur Herstellung von pharmazeutischen Präparaten.

14. Die Verwendung der neuen Verbindungen der Formel I zur Behandlung von Erkrankungen des rheumatoiden Formenkreises.

15. Verfahren zur Herstellung neuer Verbindungen der Formel I gemäss Anspruch 1, dadurch gekennzeichnet, dass man

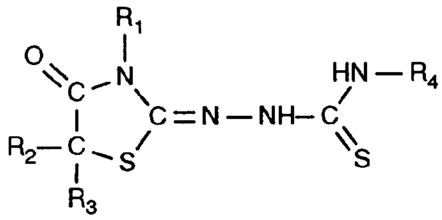

(II)

mit einer entsprechenden Verbindung der Formel III

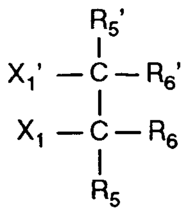

(III

worin $X_1$ und $X_1$' jeweils ein reaktionsfähiges verestertes Hydroxy bedeuten und $R_5$, $R_6$, $R_5$' und $R_6$' die oben angegebenen Bedeutungen haben, in Gegenwart eines basischen Kondensationsmittel unter nachfolgendem Erwärmen kondensiert und gewünschtenfalls ein erfindungsgemäss erhältliches Isomerengemisch in die individuellen Isomeren auftrennt und das gewünschte Isomere isoliert und/oder eine erfindungsgemäss erhältliche freie Verbindung in ein Salz oder ein erfindungsgemäss erhältliches Salz in die freie Verbindung oder in ein anderes Salz überführt.

Europäisches Patentamt

# EUROPÄISCHER RECHERCHENBERICHT

Nummer der Anmeldung

EP 92 81 0971

| EINSCHLÄGIGE DOKUMENTE | | | |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl.5 )** |
| A | EP-A-0 432 740 (HOECHST A.G.)<br>* Beispiel 4 * | 1-14 | C07D417/12<br>A61K31/425 |
| A | CHEMICAL ABSTRACTS, vol. 102, no. 11, 18. März 1985, Columbus, Ohio, US; abstract no. 89678x, M.G. VIGORITA ET AL.<br>* Zusammenfassung *<br>& FARMACO, ED. SCI. Bd. 39, Nr. 12, 1984, Seiten 1008 - 23 | 1-14 | |
| A | EP-A-0 069 784 (MITSUI TOATSU KAGAKU KABUSHIKI)<br>* Tabelle 1 * | 1-14 | |
| A | EP-A-0 069 154 (MITSUI TOATSU KAGAKU KABUSHIKI)<br>* Tabelle 1 * | 1-14 | **RECHERCHIERTE SACHGEBIETE (Int. Cl.5 )**<br>C07D<br>A61K |

Der vorliegende Recherchenbericht wurde für alle Patentansprüche erstellt

| Recherchenort | Abschlußdatum der Recherche | Prüfer |
|---|---|---|
| BERLIN | 04 FEBRUAR 1993 | FRELON D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)